# EUROPEAN PATENT SPECIFICATION

(11) **EP 2 094 693 B1**
(45) Date of publication and mention of the grant of the patent: **16.11.2011**
(21) Application number: 07855994.5
(22) Date of filing: 21.12.2007
(51) Int. Cl.: C07D 453/00

(54) **A METHOD FOR THE PREPARATION OF SOLIFENACIN**
VERFAHREN ZUR HERSTELLUNG VON SOLIFENACIN
PROCÉDÉ DE PRÉPARATION DE LA SOLIFÉNACINE

(30) Priority: 22.12.2006 CZ 20060828
(43) Date of publication of application: 02.09.2009
(73) Proprietor: Zentiva, k.s., Dolni Mecholupy 102 37 Praha 10 (CZ)
(72) Inventor: JIRMAN, Josef, 100 00 Praha 10 (CZ); JUNEK, Richard, 104 00 Praha 10 (CZ)
(74) Representative: Jirotkova, Ivana
(86) International application number: PCT/CZ2007/000119
(87) International publication number: WO 2008/077357

(56) References cited:
- EP-A- 0 801 067
- EP-A- 1 714 965
- JP-A- 52 108 992

## Description

### TECHNICAL FIELD

The invention deals with a new method of preparation of (1S)-(3R)-1-azabicyclo[2.2.2]oct-3-yl 3,4-dihydro-1-phenyl-2(1H)-isoquinoline carboxylate of formula III (solifenacin), which is used for symptomatic treatment of urgent incontinence and/or increased frequency of urinating and urgency of urinating in patients with hyperactive urinary bladder.

### BACKGROUND ART

The original patent literature (EP 0 801 067, WO 9620194) describes several methods of preparation of solifenacin.
The first method consists in the reaction of (1S)-ethyl 1-phenyl-1,2,3,4-tetrahydro-2-isoquinoline carboxylate of formula II with 3-(R)-quinuclidol of formula I in a toluene suspension and in the presence of sodium hydride. The reaction mixture is refluxed with simultaneous distillation of the ethanol being formed, which leaves in the form of an azeotropic mixture with toluene.

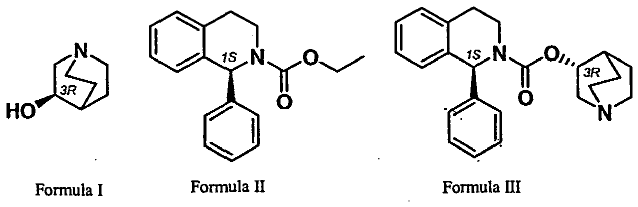

Another method of production described in the patent is the reaction of 1-phenyl-1,2,3,4-tetrahydro-2-isoquinoline carbonyl chloride of formula IV with 3-quinuclidol of formula V in dimethylformamide, producing 1-azabicyclo[2.2.2.]oct-3-yl 3,4-dihydro-1-phenyl-2(1H)-isoquinoline carboxylate of formula VI.

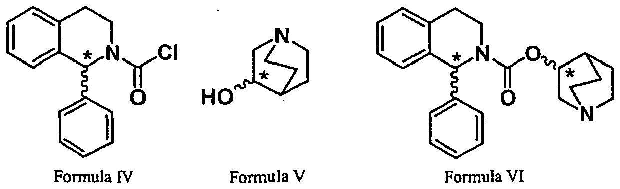

In EP 0 801 067, or WO 9620194 another version is published, where 3-(R)-quinuclidyl alkyl carbonate is reacted with 1-phenyl-1,2,3,4-tetrahydro-2-isoquinoline to produce solifenacin. Crude solifenacin obtained with the use of one of the above mentioned methods of preparation is subsequently isolated as the hydrochloride or oxalate and during their crystallization purification from unreacted starting materials and side products occurs. However, the purifying effect of the transformation to these salts is small. To achieve purity that is suitable for the preparation of the end product, solifenacin succinate, which is used for the preparation of medicaments, the crystallization of HCl or oxalate must be repeated several times, which naturally reduces the yields.
EP 1 714 965 discloses a method for reducing the impurities of solifenacin by preparing the succinate salt. Document JP 52 108992 uses tartaric acid in the purification of cefalexin.

### DISCLOSURE OF INVENTION

The invention provides a new method of preparation of optically pure (1S)-(3R)-1-azabicyclo[2.2.2]oct-3-yl 3,4-dihydro-1-phenyl-2(1H)-isoquinoline carboxylate (solifenacin) or its pharmaceutically acceptable salts, which consists in the preparation of solifenacin hydrogen tartrate in a crystalline form. The invention is based on the exceptionally good purification effect of the transformation of the crude base to the hydrogen tartrate by the action of L-tartaric acid and subsequent isolation of crystalline solifenacin hydrogen tartrate. The method of the invention can be used for purification of solifenacin prepared by any known method of preparation, in particular for solifenacin prepared by reaction of (1S)-alkyl 1-phenyl-1,2,3,4-tetrahydro-2-isoquinoline carboxylate with s 3-(R)-quinuclidol with catalysis of a non-nucleophilic base.
Solifenacin hydrogen tartrate has not been prepared and characterized so far. Crystalline solifenacin hydrogen tartrate is a stable salt that can be transformed to another pharmaceutically acceptable solifenacin salt, e.g. succinate, or used directly for the preparation of a medicament.
This salt has been characterized by the X-ray, DSC, IR and CP-MAS ¹³C NMR spectroscopy methods.
The method of the invention can be carried out, e.g., in the following manner:
A reaction mixture with crude solifenacin, prepared by any known method (see above) is cooled after the achievement of the conversion and water is added to the mixture. After separation of the organic phase the aqueous layer is washed with another portion of the organic solvent. The combined organic extracts are washed with water, saline and finally with water again. The organic layer is subsequently evaporated to dryness.
After this purification some impurities still remain in the reaction mixture, such as (1R)-(3R)-1-azabicyclo[2.2.2]oct-3-yl 3,4-dihydro-1-phenyl-2(1H)-isoquinoline carboxylate, (1S)-(3S)-1-azabicyclo[2.2.2]oct-3-yl 3,4-dihydro-1-phenyl-2(1H)-isoquinoline carboxylate and 1(S)-phenyl-1,2,3,4-tetrahydroisoquinoline.

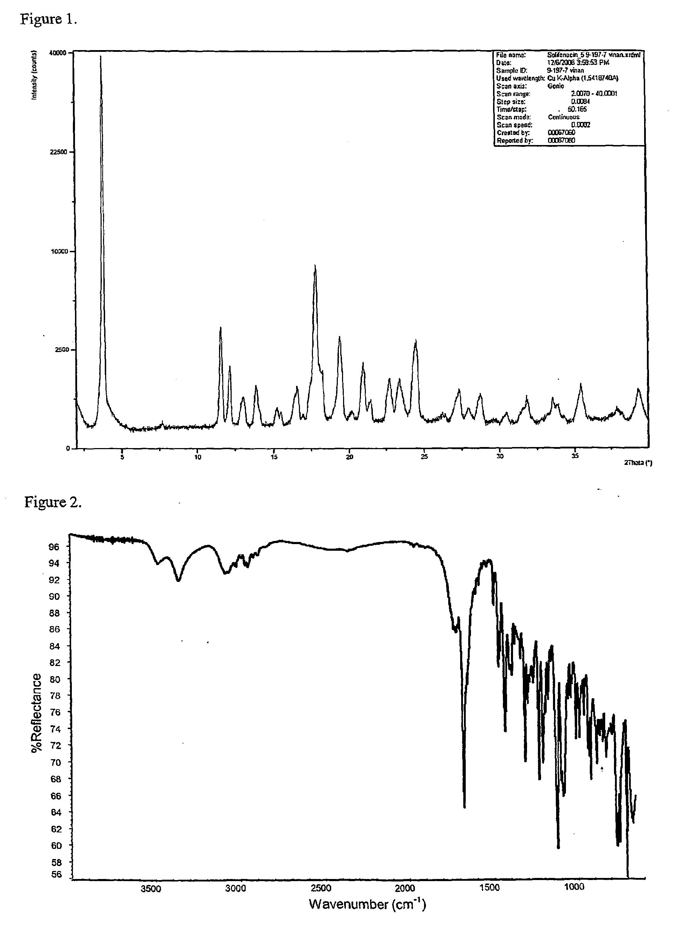

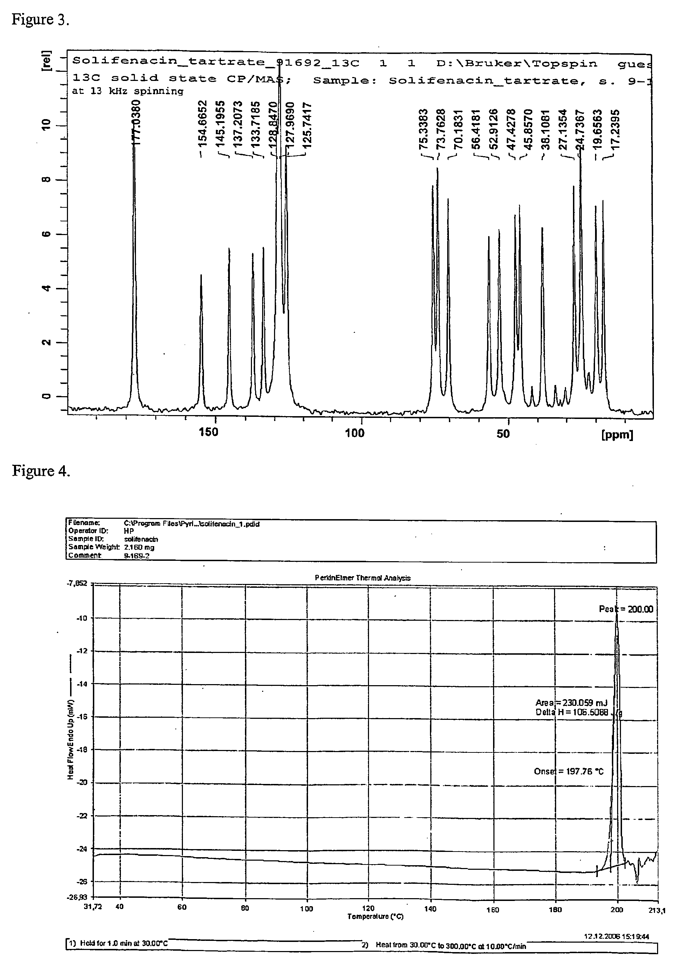

As purification of solifenacin by its crystallization in the form of the HCl salt or oxalate has manifested low efficiency, there was an effort to find a more suitable way of purification of crude solifenacin.
What has proved to be more suitable was to convert the solifenacin base to the succinate by the action of succinic acid. However, by far the best cleaning effect was achieved with the conversion of the crude solifenacin base to the hydrogen tartrate crystalline salt by the action of L-tartaric acid.
The crystalline form of the hydrogen tartrate of (1S)-(3R)-1-azabicyclo[2.2.2]oct-3-yl 3,4-dihydro-1-phenyl-2(1H)-isoquinoline carboxylate has been characterized by means of the X-ray diffraction pattern method (signals at 3.9 , 11.6, 12.1 , 13.9, 17.8 , 19.5 and 24.5 ± 0.2 degrees 2θ), IR and CP-MAS ¹³C NMR spectroscopies and DSC (1 peak at 200.0 °C) (Figures 1 to 4).

The process that has proved to represent the most efficient way of preparation of solifenacin is the process comprising: the preparation of the solifenacin base by reaction of (1S)-alkyl 1-phenyl-1,2,3,4-tetrahydro-2-isoquinoline carboxylate with 3-(R)-quinuclidol with catalysis by a non-nucleophilic base (Scheme 1), 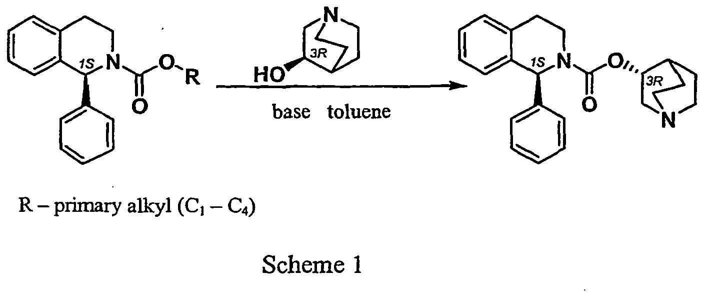 further the isolation of the crude base and subsequent final purification, which consists in transforming the solifenacin base to the hydrogen tartrate by the action of L-tartaric acid in the environment of a polar solvent or mixture of polar solvents, and isolation of crystalline solifenacin hydrogen tartrate.
The solifenacin hydrogen tartrate pure salt prepared this way can be transformed to pharmaceutically commonly used solifenacin succinate, or directly used for the preparation of a medicament.

A non-nucleophilic base is a base having considerably reduced capability of nucleophilic substitution. This is mainly the case of sterically hindered alcoholates or amines, further of lithium compounds or of the group of substances called phosphazenes, in particular e.g. potassium tert-butoxide, sodium tert-butoxide, tert-butyllithium, LDA, K-HMDS, DBU, DBN, 2,6-di-tert-butyl-4-methylpyridine, P1-t-Bu base, BEMP, BTPP and P2-t-Bu base while potassium tert-butoxide and sodium or potassium tert-amylate are especially suitable.
From the group of polar solvents for the preparation and isolation of hydrogen tartrate it is most suitable to use C₁-C₄ alcohols, their mixtures and possibly their combinations with water.

Solifenacin tartrate can be alternatively prepared in such a way that L-tartaric acid is added directly to the organic phase with the crude solifenacin base, i.e. without prior isolation of the base, e.g. in such a way that an equivalent of the saturated solution of L-tartaric acid in an alcohol is added to the solution of the crude base in a non-polar solvent. Solifenacin hydrogen tartrate is precipitated, sucked off and subsequently crystallized in a usual way.

### BRIEF DESCRIPTION OF DRAWINGS

Figure 1 represents an XRPD diffraction pattern of (1S)-(3R)-1-azabicyclo[2.2.2]oct-3-yl 3,4-dihydro-1-phenyl-2(1H)-isoquinoline carboxylate hydrogen tartrate.
Figure 2 represents an IR spectrum of (1S)-(3R)-1-azabicyclo[2.2.2]oct-3-yl 3,4-dihydro-1-phenyl-2(1H)-isoquinoline carboxylate hydrogen tartrate.
Figure 3 represents a CP-MAS ¹³C NMR spectrum of (1S)-(3R)-1-azabicyclo[2.2.2]oct-3-yl 3,4-dihydro-1-phenyl-2(1H)-isoquinoline carboxylate hydrogen tartrate.
Figure 4 represents a DSC curve of (1S)-(3R)-1-azabicyclo[2.2.2]oct-3-yl 3,4-dihydro-1-phenyl-2(1H)-isoquinoline carboxylate hydrogen tartrate

### EXAMPLES

### General procedure of examples 1-6

3(R)-Quinuclidol (0.1 - 0.15 mmol) is added to a solution of 1(*S*)-ethyl 1-phenyl-1,2,3,4-tetrahydro-2-isoquinoline carboxylate (0.1 mmol) in toluene (25 ml). After heating to 90 °C and complete dissolution of both starting substances potassium tert-butanolate (0 - 0.05 mmol) is added to the mixture during continuous stirring and the resulting fine suspension is heated up to boil. The azeotropic mixture toluene - alcohol is gradually removed from the mixture by distillation. The reaction is completed after the achievement of the corresponding conversion.

**The course of the reaction (substance conversion) was monitored with gas chromatography.**
**The optical purity of the product was determined with capillary electrophoresis.**
**The results of analyses of the products prepared in accordance with examples 1- 6 are summarized in the table.**

| **Batch** | **Charge (%)** | | **3 hours (%)** | | | **4 hours (%)** | | | **5 hours (%)** | | | **6 hours (%)** | | | **10 hours (%)** | | |
|---|---|---|---|---|---|---|---|---|---|---|---|---|---|---|---|---|---|
| | Chin | tBOK | conv. | *R,R* | *S,S* | conv. | *R,R* | *S,S* | conv. | *R,R* | *S,S* | conv. | *R,R* | *S,S* | conv. | *R,R* | *S,S* |
| **9-142** | 120 | 20 | 96,11 | 1,77 | 1,97 | 96,45 | 2,07 | 2,27 | 97.07 | 2,27 | 2,27 | - | - | - | - | - | - |
| **9-143** | 110 | 10 | 51,29 | 0,21 . | 0,50 | 78,20 . | 1,32 | 0,78 | 87,45 | 1.82 | 0.951 | 95,86 | 5,12 | 1,42 | 96,07 | 5,13 | 1,51 |
| **9-144** | 110 | 20 | 96,80 | 5,12 | 2,33 | - | - | - | - | - | - | - | - | - | - | - | - |
| **9-145** | 130 | 20 | 87,52 | 2,15 | 0,78 | - | - | - | - | - | - | - | - | - | - | - | - |
| **9-146** | 120 | 30 | 96,01 | 4,56 | 1.38 | - | - | - | - | - | - | - | - | - | - | - | - |
| **9-148** | 130 | 10 | 34,53 | 0,00 | 0,00 | - | - | - | 64,93 | 0,40 | 0,48 | 76,45 | 0,58 | 0,75 | - | - | - |

### Example 7:

4 g of the solifenacin base prepared in accordance with procedure 6, which contained 4.5 % of (1R)-(3R)-1-azabicyclo[2.2.2.]oct-3-yl 3,4-dihydro-1-phenyl-2(1H)-isoquinoline carboxylate, 1.4 % of (1S)-(3S)-1-azabicyclo[2.2.2.]oct-3-yl 3,4-dihydro-1-phenyl-2(1H)-isoquinoline carboxylate and 4.0 % of 1(S)-phenyl-1,2,3,4-tetrahydroisoquinoline were dissolved in 30 ml of methylethylketone. An equivalent of a 3M solution of hydrogen chloride in methanol was added to the solution and the mixture was left to freely crystallize at the room temperature. After sucking off and washing, 42% of solifenacin hydrochloride was obtained, which contained 1.7 % of (1R)-(3R)-1-azabicyclo[2.2.2.]oct-3-yl 3,4-dihydro-1-phenyl-2(1H)-isoquinoline carboxylate hydrochloride, 0.6 % of (1S)-(3S)-1-azabicyclo[2.2.2.]oct-3-yl 3,4-dihydro-1-phenyl-2(1H)-isoquinoline carboxylate hydrochloride and 1.3 % of 1(S)-phenyl-1,2,3,4-tetrahydroisoquinoline hydrochloride. In crystallizing the product from methylethylketone 58 % of solifenacin hydrochloride was obtained, which contained 0.9 % of (1R)-(3R)-1-azabicyclo[2.2.2.]oct-3-yl 3,4-dihydro-1-phenyl-2(1H)-isoquinoline carboxylate hydrochloride, 0.4 % of (1S)-(3S)-1-azabicyclo[2.2.2.]oct-3-yl 3,4-dihydro-1-phenyl-2(1H)-isoquinoline carboxylate hydrochloride and 0.7% of 1(S)-phenyl-1,2,3,4-tetrahydroisoquinoline hydrochloride. After the second crystallization from methylethylketone 63 % of solifenacin hydrochloride was obtained, which contained 0.4 % of (1R)-(3R)-1-azabicyclo[2.2.2.]oct-3-yl 3,4-dihydro-1-phenyl-2(1H)-isoquinoline carboxylate hydrochloride, 0.18 % of (1S)-(3S)-1-azabicyclo[2.2.2.]oct-3-yl 3,4-dihydro-1-phenyl-2(1H)-isoquinoline carboxylate hydrochloride and 0.3% of 1(S)-phenyl-1,2,3,4-tetrahydroisoquinoline hydrochloride.

### Example 8:

3(*R*)-Quinuclidol (44.3 mmol) was added to a solution of 1(*S*)-ethyl 1-phenyl-1,2,3,4-tetrahydro-2-isoquinoline carboxylate (36.7 mmol) in 250 ml of toluene. After heating to 90 °C and complete dissolution of both starting substances potassium tert-amylate (7.4 mmol) was added to the mixture during continuous stirring and the resulting fine suspension was heated up to boil. The azeotropic mixture toluene - alcohol was gradually removed from the mixture by distillation. The reaction was finished after 3 hours of boiling, when the conversion in accordance with GC achieved 96%. Water (100 ml) was added to the mixture and the mixture was stirred at the laboratory temperature for 30 minutes. After the separation of the toluene layer in a separator the aqueous layer was washed with toluene (2x 100 ml). The combined organic extracts were then further extracted with 150 ml of diluted hydrochloric acid (6.1 mmol) and the toluene layer was then washed with water (2x 50ml). Then, the aqueous extracts were alcalinized with potassium carbonate (10.9 mmol) and extracted with toluene (3x 100ml). The organic extracts were washed with 100 ml of water, 50 ml of saline, 50 ml of water and evaporated in a rotational vacuum evaporator 12.7 g of the solifenacin base was obtained that contained the following impurities (CE analysis).
3.2 % of (1R)-(3R)-1-azabicyclo[2.2.2.]oct-3-yl 3,4-dihydro-1-phenyl-2(1H)-isoquinoline carboxylate, 1.1 % of (1S)-(3S)-1-azabicyclo[2.2.2.]oct-3-yl 3,4-dihydro-1-phenyl-2(1H)-isoquinoline carboxylate and 6.0 % of 1(S)-phenyl-1,2,3,4-tetrahydroisoquinoline.

### Example 9:

One half of the solifenacin base prepared in accordance with procedure 8 was dissolved in 50 ml of a methanol:2-propanol (1:1) mixture, an equivalent of L-tartaric acid in a methanol:2-propanol (1:1) mixture was added and the mixture was left to freely crystallize at the room temperature. After sucking off and washing, 79 % of solifenacin hydrogen tartrate was obtained, which contained 1.1 % of (1R)-(3R)-1-azabicyclo[2.2.2.]oct-3-yl 3,4-dihydro-1-fenyl-2(1H)-isoquinoline carboxylate hydrogen tartrate, 0.6 %,of (1S)-(3S)-1-azabicyclo[2.2.2.]oct-3-yl 3,4-dihydro-1-phenyl-2(1H)-isoquinoline carboxylate hydrogen tartrate and no 1(S)-phenyl -1,2,3,4-tetrahydroisoquinoline. In crystallizing the product from the methanol:2-propanol (3:4) mixture 94 % of solifenacin hydrogen tartrate was obtained, which contained 0.6% of (1R)-(3R)-1-azabicyclo[2.2.2.]oct-3-yl 3,4-dihydro-1-fenyl-2(1H)-isoquinoline carboxylate hydrogen tartrate and 0.4 % of (1S)-(3S)-1-azabicyclo[2.2.2.]oct-3-yl 3,4-dihydro-1-phenyl-2(1H)-isoquinoline carboxylate hydrogen tartrate. After the second crystallization from ethanol 89% of solifenacin hydrogen tartrate was obtained, which did not contain any detectable impurities. The product was characterized by means of the following analytical methods: X-ray, IR and CP-MAS ¹³C NMR spectroscopies and DSC.

### Example 10:

The other half of the solifenacin base prepared in accordance with procedure 8 was dissolved in 15 ml of 2-propanol, an equivalent of succinic acid in 55 ml of 2-propanol was added and the mixture was left to freely crystallize at the room temperature. After sucking off and washing, 66% of solifenacin succinate was obtained, which contained 1.0% of (1R)-(3R)-1-azabicyclo[2.2.2.]oct-3-yl 3,4-dihydro-1-phenyl-2(1H)-isoquinoline carboxylate succinate, 0.7 % of (1S)-(3S)-1-azabicyclo[2.2.2.]oct-3-yl 3,4-dihydro-1-phenyl-2(1H)-isoquinoline carboxylate succinate and 0.3 % of 1(S)-phenyl-1,2,3,4-tetrahydroisoquinoline. In crystallizing the product from 2-propanol 86% of solifenacin succinate was obtained, which contained 0.41% of (lR)-(3R)-1-azabicyc10[2.2.2.]oct-3-yl 3,4-dihydro-1-phenyl-2(1H)-isoquinoline carboxylate succinate, 0.35 % of (1S)-(3S)-1-azabicyclo[2.2.2.]oct-3-yl 3,4-dihydro-1-phenyl-2(1H)-isoquinoline carboxylate succinate and 0.1 % of 1(S)-phenyl-1,2,3,4-tetrahydroisoquinoline. After the second crystallization from 2-propanol 89% of solifenacin succinate was obtained, which contained 0.17 % (1R)-(3R)-1-azabicyclo[2.2.2.]oct-3-yl 3,4-dihydro-1-phenyl-2(1H)-isoquinoline carboxylate succinate, 0.33 % of(1S)-(3S)-1-azabicyclo[2.2.2.]oct-3-yl 3,4-dihydro-1-phenyl-2(1H)-isoquinoline carboxylate succinate and 0.07 % of 1(S)-phenyl-1,2,3,4-tetrahydroisoquinoline.

## Claims

1. A method for the preparation of (1S)-(3R)-1-azabicyclo[2.2.2]oct-3-yl 3,4-dihydro-1-phenyl-2(1H)-isoquinoline carboxylate (solifenacin) or its pharmaceutically acceptable salts with high optic purity, **characterized in that** the crude solifenacin base is converted to the hydrogen tartrate, which is then optionally converted to another pharmaceutically acceptable salt or the base of solifenacin.

2. The method in accordance with claim 1, **characterized by** in that the solifenacin base is converted to the hydrogen tartrate by the action of L-tartaric acid and crystalline solifenacin hydrogen tartrate is subsequently isolated.

3. The method in accordance with claim 2, **characterized in that** solifenacin hydrogen tartrate is further re-crystallized from a polar solvent or a mixture of polar solvents, preferably from the group of C₁-C₄ alcohols, or their combination with water.

4. The method in accordance with claims 2 and 3, **characterized in that** the crude solifenacin base is prepared by reaction of (1S)-alkyl 1-phenyl-1,2,3,4-tetrahydro-2-isoquinoline carboxylate, where alkyl means a primary C₁-C₄ alkyl, with 3-(R)-quinuclidol in the environment of a non-nucleophilic base.

5. The method in accordance with claim 4, **characterized in** fact that a non-nucleophilic base from the group of sterically hindered alcoholates or amines, or lithium compounds, or phosphazenes, is used.

6. The method in accordance with claim 5, **characterized in that** potassium, sodium or lithium tert-butanolate is used.

7. The method in accordance with claim 5, **characterized in that** potassium or sodium tert-amylate is used.

8. A crystalline salt of solifenacin hydrogen tartrate.

9. The crystalline salt in accordance with claim 8, **characterized by** the X-ray spectrum record with typical signals at: 3.9, 11.6, 12.1, 13.9, 17.8, 19.5 and 24,5 ± 0,2 degrees 2θ.

10. The crystalline salt in accordance with claim 8, **characterized by** the DSC record with 1 endotherm with the peak at 200.0 °C at the heating speed of 10 °C/min.

11. The crystalline salt in accordance with claim 8, **characterized by** the signals at 11.2 19.6 24.7 27.1 38.1 45.8 47.4 52.9 56.4 70.1 73.7 75.3 125.7 127.9 128.8 133.7 137.2 145.2 154.6 177.0 ppm in the CP/13C MAS NMR spectrum.

## Patentansprüche

1. Verfahren zur Herstellung von (1S)-(3R)-1-Azabicyclo[2.2.2]oct-3-yl-3,4-dihydro-1-phenyl-2(1H)-isochinolincarboxylat (Solifenacin) oder dessen pharmazeutisch akzeptablen Salzen mit einer hohen optischen Reinheit, welches **dadurch gekennzeichnet ist, dass** die rohe Solifenacinbase in das Hydrogentartrat umgewandelt wird, das anschließend wahlweise in ein weiteres pharmazeutisch akzeptables Salz oder in die Base des Solifenacins umgewandelt wird.

2. Verfahren gemäß Anspruch 1, welches **dadurch gekennzeichnet ist, dass** die Solifenacinbase durch die Wirkung von L-Weinsäure in das Hydrogentartrat umgewandelt wird und anschließend kristallines Solifenacinhydrogentartrat isoliert wird.

3. Verfahren gemäß Anspruch 2, welches **dadurch gekennzeichnet ist, dass** das Solifenacinhydrogentartrat weiterhin aus einem polaren Lösungsmittel oder einer Mischung aus polaren Lösungsmitteln, vorzugsweise aus der Gruppe der C₁-C₄-Alkohole, oder deren Kombinationen mit Wasser umkristallisiert wird.

4. Verfahren gemäß Anspruch 2 oder 3, welches **dadurch gekennzeichnet ist, dass** die rohe Solifenacinbase durch Reaktion von (1S)-Alkyl-1-phenyl-1,2,3,4-tetrahydro-2-isochinolincarboxylat mit 3-(R)-Chinuclidol in der Umgebung einer nicht-nukleophilen Base hergestellt wird, worin Alkyl ein primäres C₁-C₄-Alkyl bedeutet.

5. Verfahren gemäß Anspruch 4, welches **dadurch gekennzeichnet ist, dass** eine nicht-nukleophile Base aus der Gruppe der sterisch gehinderten Alkoholate oder Amine, oder Lithiumverbindungen oder Phosphazene verwendet wird.

6. Verfahren gemäß Anspruch 5, welches **dadurch gekennzeichnet ist, dass** Kalium, Natrium oder Lithium tert-Butanolat verwendet wird.

7. Verfahren gemäß Anspruch 5, welches **dadurch gekennzeichnet ist, dass** Kalium oder Natrium tert-Amylat verwendet wird.

8. Kristallines Salz des Solifenacinhydrogentartrats.

9. Kristallines Salz gemäß Anspruch 8, welches **dadurch gekennzeichnet ist, dass** das Röntgenspektrum ein typisches Signal bei 3,9, 11,6, 12,1, 13,9, 17,8, 19,5 und 24,5 ± 0,2 Grad 2θ aufzeichnet.

10. Kristallines Salz gemäß Anspruch 8, welches durch die DSC-Aufzeichnung mit 1 Endothermen mit dem Peak bei 200,0°C bei einer Heizgeschwindigkeit von 10°c/min gekennzeichnet ist.

11. Kristallines Salz gemäß Anspruch 8, welches durch die Signale bei 11,2, 19,6, 24,7, 27,1, 38,1, 45,8, 47,4, 52,9, 56,4, 70,1, 73,7, 75,3, 125,7, 127,9, 128,8, 133,7, 137,2, 145,2, 154,6, 177,0 ppm in dem CP/13C MAS NMR-Spektrum gekennzeichnet ist.

## Revendications

1. Un procédé pour la préparation de carboxylate de (1S)-(3R)-1-azabicyclo[2.2.2]oct-3-yl 3,4-dihydro-1- phényl-2(1H)-isoquinoline (solifénacine) ou de ses sels pharmaceutiquement acceptables présentant une pureté optique élevée, **caractérisé en ce que** la base brute de solifénacine est transformée en hydrogéno-tartrate, qui est alors éventuellement transformé en un autre sel pharmaceutiquement acceptable ou la base de solifénacine.

2. Le procédé selon la revendication 1, **caractérisé en ce que** la base de solifénacine est transformée en hydrogéno-tartrate par action de l'acide L-tartrique et **en ce que** le tartrate acide cristallin de solifénacine est ultérieurement isolé.

3. Le procédé selon la revendication 2, **caractérisé en ce que** l'hydrogénotertrate de solifénacine est en outre re-cristallisé dans un solvant polaire ou un mélange de solvants polaires, de préférence dans le groupe des alcools en C₁-C₄ ou de leur combinaison avec de l'eau.

4. Le procédé selon la revendication 2 ou 3, **caractérisé en ce que** la base brute de solifénacine est préparée par réaction de carboxylate de (1S)-alkyl 1-phényl-1,2,3,4-tétrahydro-2- isoquinoline, où par alkyl on entend un radical alkyl primaire en C₁-C₄, avec du 3-(R)-quinuclidol dans un milieu formé d'une base non-nucléophile.

5. Le procédé selon la revendication 4, **caractérisé en ce qu'**est employée une base non-nucléophile du groupe des amines ou des alcoolates stériquement encombrés, des composés à base de lithium ou des phosphazènes.

6. Le procédé selon la revendication 5, **caractérisé en ce qu'**est employé du tert-butanolate de potassium, de sodium ou de lithium.

7. Le procédé selon la revendication. 5, **caractérisé en ce qu'**est employé du tert-amylate de potassium ou de sodium.

8. Un sel cristallin d'hydrogéno-tartrate de solifénacine.

9. Le sel cristallin selon la revendication 8, **caractérisé par** un enregistrement du spectre de rayons-X présentant les signaux caractéristiques suivants: 3,9, 11,6, 12,1, 13,9, 17,8, 19,5 et 24,5 ± 0,2 degrés 2θ.

10. Le sel cristallin selon la revendication 8, **caractérisé par** un enregistrement DSC présentant 1 endotherme ayant un pic à 200,0°C à une vitesse de chauffage de 10°C/min.

11. Le sel cristallin selon la revendication 8, **caractérisé par** les signaux à 11,2; 19,6; 24,7; 27,1; 38,1; 45,8; 47,4; 52,9; 56,4; 70,1; 73,7; 75,3 125,7; 127,9; 128,8; 133,7; 137,2; 145,2; 154,6; 177,0 ppm dans le spectre RMN CP/13C MAS.
